# EUROPEAN PATENT SPECIFICATION

(11) **EP 2 920 295 B1**
(45) Date of publication and mention of the grant of the patent: **11.01.2017**
(21) Application number: 13815578.3
(22) Date of filing: 13.11.2013
(51) Int. Cl.: C12M 1/00

(54) **READY-TO-USE DEVICE AND METHOD FOR REMOVING INTERFERING FACTORS FROM SAMPLES TO BE SUBJECTED TO MICROBIOLOGICAL EXAMINATION**
GEBRAUCHSFERTIGE VORRICHTUNG ZUR ENTFERNUNG STÖRENDER FAKTOREN AUS PROBEN VOR EINER MIKROBIOLOGISCHEN UNTERSUCHUNG
DISPOSITIF PRÊT À L'EMPLOI ET PROCÉDÉ POUR ÉLIMINER LES FACTEURS D'INTERFÉRENCE D'ÉCHANTILLONS À SOUMETTRE À UN EXAMEN MICROBIOLOGIQUE

(30) Priority: 15.11.2012 IT VR20120229
(43) Date of publication of application: 23.09.2015
(73) Proprietor: AL.CHI.MI.A. S.r.l., 35020 Ponte San Nicolò (PD) (IT)
(72) Inventor: BECCARO, Mauro, I-35010 Cadoneghe (IT); GATTO, Claudio, I-31050 Merlengo di Ponzano Veneto(TV) (IT); D'AMATO TÓTHOVÁ, Jana, I-30030 Pianiga (VE) (IT); BETTINI, Enrico, I-30032 Fiesso D'Artico (Venezia) (IT); SIGNORI, Paolo, I-37131 Verona (VR) (IT)
(74) Representative: Marchi, Paolo
(86) International application number: PCT/IB2013/060097
(87) International publication number: WO 2014/076640

(56) References cited:
- US-A- 4 145 304
- US-A1- 2004 120 942
- US-A1- 2006 167 380
- MORTEZA ABBASZADEGAN ET AL: "Detection of Enteroviruses in Groundwater with the Polymerase Chain Reaction", APPLIED AND ENVIRONMENTAL MICROBIOLOGY, 1 May 1993 (1993-05-01), pages 1318-1324, XP055076740,
- "International Congress of the European Association of Tissue Banks (EATB) Venue Langenbeck-Virchow-Haus Congress President", , 1 November 2010 (2010-11-01), XP055103853, Retrieved from the Internet: URL:http://www.kongress-server.de/fileadmi n/media/eatb/Programm_EATB__WEB_.pdf [retrieved on 2014-02-24]
- ABOLMAATY ET AL: "The use of activated charcoal for the removal of PCR inhibitors from oyster samples", JOURNAL OF MICROBIOLOGICAL METHODS, ELSEVIER, AMSTERDAM, NL, vol. 68, no. 2, 27 January 2007 (2007-01-27), pages 349-352, XP005863297, ISSN: 0167-7012, DOI: 10.1016/J.MIMET.2006.09.012

## Description

This invention relates to a ready-to-use device and a method for removing interfering factors from samples to be subjected to microbiological examination.

In the context of this invention, microbiological examination means any examination/test intended either to verify the microbiological negativity of a sample, or to measure its degree and type of microbiological contamination. Microbiological negativity is usually required for organs, tissues or cells intended for transplantation whose effective implantability must be checked, and for a medicine to be put on sale, or for a substance derived from humans which is also intended to be administered to a patient. In fact, in all of these cases, the absence of microbiological negativity could result in serious consequences for the patient.

In contrast, the check of the degree and type of microbiological contamination is usually performed in the case of laboratory examinations of bodily fluids taken from a person, where the aim is not to check for the existence of a sterile condition, but rather to check whether or not there is an infection present.

The methods currently used for microbiological examinations are generally based on bacterial reproduction. In fact, the sample to be examined is placed in a culture environment able to promote bacterial reproduction which causes the formation of substances which are then detected by suitable equipment (for example, in many applications what is detected is an increased degree of cloudiness of the culture liquid or the production of gas, in most cases carbon dioxide, by means of detection of a change of colour/luminescence due to chromogenic agents which are sensitive to the gas).

It is easy to infer how the results of such analyses may be distorted if, despite the presence of microbial contaminations, conditions arise which prevent normal bacterial reproduction. In particular, it is known that a bacterial contamination present may not be detected if the sample being examined is also contaminated by one or more substances which act as interfering factors. In fact, the term "interfering factor" identifies any substance able to inhibit or slow bacterial reproduction and it will be used with that meaning hereinafter in this description. Therefore, that definition includes both bactericidal agents such as antibiotics, and bacteriostatic agents such as disinfectants.

However, in the context of the main applications of this invention, the most common interfering factor is antibiotics. Such substances on one hand may be present in an organism from which an organ, a tissue, cells or fluids are taken (for example due to an antibiotic treatment to which the subject was previously subjected), and on the other hand are always present in the liquids used for the decontamination and preservation of tissues, organs and cells intended for transplantation.

The problem of interfering factors is more evident in the case of checks of the sterility of medicines, since the medicine itself usually acts as an interfering factor.

Consequently, in order to be able to consider the result of a microbiological examination reliable, one must be certain that the sample examined is not contaminated by interfering factors, that is to say, the sample examined must be subjected to a process to purify it by removing any interfering factors from it.

Microbiological checks of bodily fluids (including blood and urine) are usually carried out using automated equipment in which the sample to be examined is inserted in a vial containing a suitable culture broth. The various known automated equipment includes the BacT/ALERT® produced and marketed by BioMerieux SA with registered office in Marcy l'Etoile, France, and the BACTEC™ produced and marketed by BD with registered office in Franklin Lakes, NJ, USA. In order to attempt to overcome the problem of interfering factors, both of these pieces of equipment require that the sample to be analysed is inserted in a vial which at the same time contains both a culture liquid for the micro-organisms and polymeric resins for inhibiting the action of any interfering factors present. When the sample has been in the vial for a predetermined period of time, the microbiological examination is performed by checking for any increase in the quantity of gas (carbon dioxide) present compared with at the start of the examination. Examples of the use of resins for removing interfering factors are also described in patents US 4632902, US 5624814, EP 73089, US 4174277, US 4145304, US 5162229, US 2009/123960, US 2011/312021 and US 5314855. The main problem with this type of examination/equipment is low sensitivity. In fact, it is known that in order to be able to check for the effective presence of a contamination, the number of bacteria (or rather of colony forming units - CFU) present in the vial must be at least equal to a predetermined minimum value. Below that threshold detection is impossible and the test result is a false negative. Moreover, as already indicated, tests run on blood and urine do not aim to check for microbiological negativity, but rather to check that the level of contamination is not greater than a threshold value scientifically acknowledged to be discriminating in order to be able to diagnose the existence of an infection. Consequently, in reality, the known equipment can provide good clinical results provided that the blood/urine sample available is large enough.

However, regarding blood tests, it should be noticed that the quantity of blood required by the equipment may be negligible if taken from an adult, but may be significant if taken from a new born baby or in any case a child (especially if several samples are required for several tests).

The matter of checking for microbiological negativity is different and more critical, especially with reference to the transplant sector, to which this invention is particularly dedicated (since this invention is preferably intended for the transplant sector, hereinafter reference will preferably be made to that sector, although, if applicable, all assessments expressed shall also be considered valid for any other possible application of this invention). In the transplant sector, ensuring that an organ, a tissue or cells to be transplanted in a patient are free of contamination is essential in order to reduce the risks linked to this type of surgery. In addition to the possible risks immediately obvious, such as the onset of infections in the transplant zone (with the risk of rejection phenomena), a contamination could in fact also bring risks of the development of infections in other sites not directly linked to the surgery. Once they are in the circulatory system any bacteria spread throughout the body and may therefore reach any site suitable for their proliferation (this phenomenon cannot be ruled out as being the basis of many infections apparently unrelated to the surgery which affect transplant recipients). Those responsible for guaranteeing microbiological negativity of organs, tissues and cells are firstly transplant centres for organs, and, for tissues and cells, the relative banks. Therefore, they have accurate microbiological examinations for each organ, tissue or set of cells to be implanted. In particular, at present microbiological examinations on tissues, organs and cells intended for transplantation may be carried out either on a sample of what is to be implanted (or on what has been implanted in the case of postoperative checks), or on a sample of the preservation/decontamination liquid in which the organ, tissue or cells were preserved before being implanted (most tissues and cells intended for implantation undergo a decontamination process by immersion in a decontamination liquid which includes a plurality of broad-spectrum antibiotics).

The main problem of this sector is that there are currently no known methods or equipment specifically intended for this type of microbiological examination (that is to say, for examinations to check for microbiological negativity). Tissue and cell banks do not even currently have a single procedure for checking for microbiological negativity.

Therefore, at present, examinations to check for microbiological negativity are usually carried out using the equipment created for blood and urine tests (such as the BacT/ALERT® and the BACTEC™ described above), by inserting in the vials the samples to be analysed in place of blood or urine. However, as already indicated, such systems were not designed to check for microbiological negativity, and they have limited sensitivity which prevents them from detecting bacterial contaminations when the ratio between the number of bacteria (CFU) and the quantity of interfering factors present falls below a predetermined minimum threshold. In fact, with the culture broths and times currently used for examinations using the known equipment, contamination is only evident when the ratio of CFU to interfering factors exceeds that minimum threshold.

Below said threshold, any bacterial multiplication which may occur is in fact insufficient to be detected in the times set. Consequently, a negative result for an examination performed using such equipment does not necessarily mean microbiological negativity. It is possible that the level of contamination of the sample is such that it cannot be detected and so gives a false negative. Although limited, the risk related even to slight contamination must not be ignored in the case of transplants.

A further problem relating to the use of equipment designed for blood and urine, for checking bacterial contamination in the transplant sector, is linked to the fact that, as already indicated, tissues and cells intended for transplantation are subjected to important decontamination processes with liquids that have a high interfering factor content. As a result, the sample examined (even if it is not the self-same decontamination liquid) contains a large quantity of interfering factors, in many cases much higher than that which could ever be present in a biological fluid such as blood or urine.

The main problem linked to this situation is the fact that the known methods and equipment are not able to eliminate/sufficiently inhibit such large quantities of interfering factors, consequently they may freely manifest their effect on any bacterial contamination present, again creating false negatives, whenever the ratio between CFU and the quantity of interfering factors is below said minimum threshold.

In particular, the known equipment is not able to eliminate/ sufficiently inhibit the interfering factors at least with the quantities of resins and in the culture times currently usable with such equipment.

Therefore, as seen, the prior art in the field of microbiological examinations has significant disadvantages.

In this context the technical purpose which forms the basis of this invention is to provide a ready-to-use device and a method for removing interfering factors from samples to be subjected to microbiological examination which overcomes the above-mentioned disadvantages.

In particular, the technical purpose of this invention is to provide a ready-to-use device and a method for removing interfering factors from samples to be subjected to microbiological examination which allows the minimising, and preferably elimination, of the risk of false negatives in checks for microbiological negativity relating to samples intended for transplantation.

A further technical purpose of this invention is to provide a ready-to-use device and a method for removing interfering factors from samples to be subjected to microbiological examination which allow an increase in the detection sensitivity of the equipment and the methods currently known.

The technical purpose specified and the aims indicated are substantially achieved by a ready-to-use device and a method for removing interfering factors from samples to be subjected to microbiological examination as described in the appended claims.

The invention provides a device for removing interfering factors from samples to be subjected to microbiological examination, said interfering factors being any substance able to inhibit or slow bacterial reproduction on which microbiological examination of the samples is based, characterized in that
the device is ready to use,
the device comprising a sealed protective envelope (2) and a containment element (3),
the containment element (3) being positioned inside the envelope (2) and containing at least one product (4) for removing interfering factors, the product (4) being in the form of granules, the particle size of the product (4) having a nominal size and a certain variation range,
the containment element (3) being closed and comprising at least one porous portion that is permeable to liquids and to the interfering factors, said at least one porous portion having a porosity with dimension less than the nominal size, such that said at least one porous portion allows the passage of particles of product (4) having dimensions less than a minimum size which is indicated as a percentage of the nominal size, and such that said at least one porous portion retains the other granules of product (4),
everything contained in the envelope (2) being sterile and the envelope (2) being such that it prevents the passage of contaminants through it in order to preserve the sterile condition of everything contained in the envelope (2), wherein the containment element (3) is extracted or sterilely removed from the envelope (2) at the time of use.

In a second aspect, the invention provides a method for removing interfering factors from a sample to be subjected to microbiological examination, said interfering factors being any substance able to inhibit or slow bacterial reproduction on which examination of the sample is based,
characterized in that it uses at least one device of the invention, the method including the steps of:
- extracting or sterilely removing the containment element (3) from the envelope (2);
- if the sample is liquid, putting the sample in fluid communication with the product (4) through the porous portion of the containment element (3) for a first period of time; or respectively
- if the sample is solid, immersing the sample in an operating liquid and putting the operating liquid in fluid communication with the product (4) through the porous portion of the containment element (3) for a second period of time.

Further features and the advantages of this invention are more apparent in the detailed description of several preferred, non-limiting embodiments of a ready-to-use device and a method for removing interfering factors from samples to be subjected to microbiological examination, with reference to the accompanying drawings, in which:
- Figure 1 is a schematic view of a first embodiment of a ready-to-use device for removing interfering factors according to this invention;
- Figure 2 is a schematic view of a second embodiment of a ready-to-use device for removing interfering factors according to this invention;
- Figure 3 is a vertical axial section of the device of Figure 2;
- Figure 4 is a schematic view of a third embodiment of a ready-to-use device for removing interfering factors according to this invention;
- Figure 5 is a vertical axial section of the device of Figure 4 with the schematic addition of an optional element.

With reference to the accompanying drawings the numeral 1 denotes in its entirety a ready-to-use device for removing interfering factors from samples to be subjected to microbiological examination, made in accordance with this invention.

In accordance with this invention, the device 1 generally comprises a sealed protective envelope 2 and a closed containment element 3 positioned inside the envelope 2. The containment element 3 contains at least one product 4 for removing interfering factors, the product being in the form of granules. Everything contained in the envelope 2 is sterile and the envelope 2 itself is such that it preserves the sterile condition, preventing the passage of contaminants.

To allow in use a free exchange of liquids and interfering factors between the inside and the outside of the containment element 3, the containment element 3 comprises at least one porous portion, made with a porosity such that it substantially retains the granules of the product 4 for removing interfering factors, but is also at the same time permeable to liquids and to the interfering factors.

Like any substance in the form of granules, the particle size of the product 4 for removing interfering factors will always have a certain variation range, for which it will always be possible to identify mean values (arithmetic or harmonic) and maximum quantities of product 4 with a size below a predetermined reference value.

Consequently, in the context of this invention, the indication that the porous portion can substantially retain the granules means that it has a porosity with dimensions less than the nominal size of the granules (understood to be the mean size), and such that it only allows the passage of particles of product 4 for removing interfering factors which have dimensions less than the minimum size (usually indicated as a percentage of the nominal size). At the same time no more than a predetermined reference quantity (% of the weight) of the product 4 may have dimensions less than the minimum size. In the preferred embodiments, the minimum size is between 50% and 85% of the nominal size, whilst the reference quantity is not greater than 10% of the weight of the product 4.

Considering that to guarantee the free passage of the liquids and any contaminants and interfering factors it is sufficient for the porous portion to have a porosity of not less than 70 µm and that optimum results may be achieved with a porosity of between 110 and 190 µm, the granules of product 4 must have a nominal size not less than 250 µm. However, in the preferred embodiments, the granules of product 4 have a nominal size (and in particular a harmonic mean size) not less than 350 µm, and a content of particles with size less than 300 µm not exceeding 10%.

The product 4 for removing interfering factors may vary depending on requirements and the intended use of the product 4.

Despite that, in the preferred embodiment (in which the device 1 is particularly suited to use related to the transplant sector), the product 4 is a composition comprising a mixture of a first substance and a second substance, both in the form of granules. The first substance in turn comprises a first resin belonging to the ion exchange resins family, whilst the second substance comprises a second resin belonging to the non-ionic hydrophobic resins family.

The first substance and the second substance are present in a ratio by weight of between 0.5 and 2, and preferably between 0.8 and 1.25 (in a particularly preferred embodiment the two substances are present in a ratio of 1:1).

Moreover, advantageously, at least the second substance is hydrated and comprises a quantity of water equal to at least 30% of its own weight, preferably equal to at least 50%. Again preferably, the second substance comprises a quantity of water not greater than 67% of its own weight. However, in the preferred embodiments, even the second substance is hydrated and comprises a quantity of water equal to at least 46% of its own weight, and advantageously not greater than 63% of its own weight. Concerning the resins present in the first and second substances, in the preferred embodiments the first resin is methacrylate-divinylbenzene based whilst the second resin is polystyrene-divinylbenzene based. Amongst those belonging to these families, good results have been obtained in particular with Amberlite™ CG50 and Amberlite™ FPC3500 resins produced by Rohm and Haas of the US group The Dow Chemical Company, as the first resin, and with the resins C18 produced and marketed by MACHEREY NAGEL, and Amberlite™ XAD™4, Amberlite™ XAD™16 and Amberlite™ XAD™18 again produced by Rohm and Haas, as the second resins. Particularly good results have been obtained with a combination of Amberlite™ FPC3500 and Amberlite™ XAD™18.

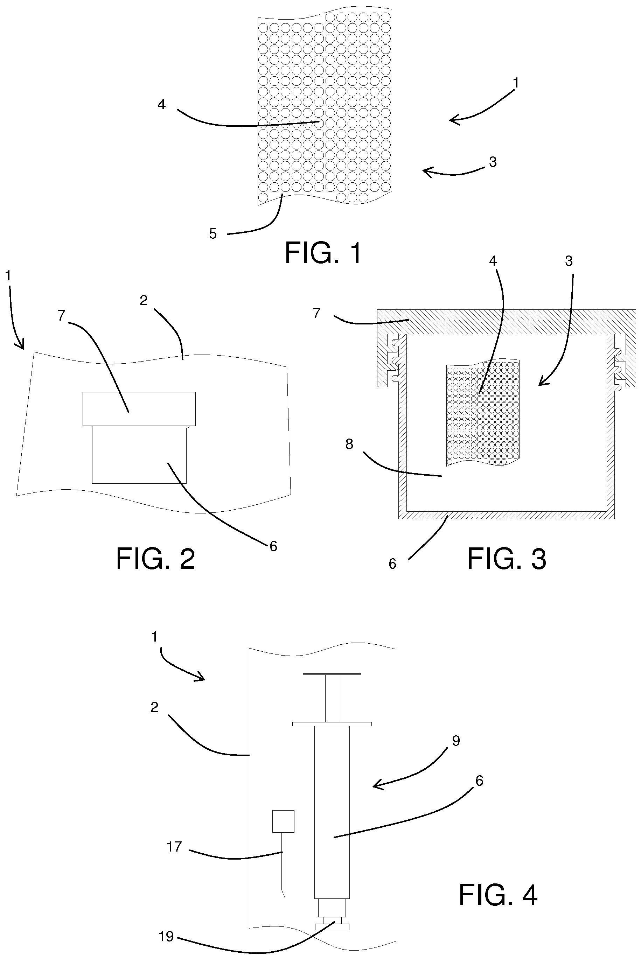

Figure 1 illustrates a first embodiment of the device 1 according to this invention, the simplest of those described herein. In this embodiment the containment element 3 is a bag 5 made partly or completely (but advantageously completely) of porous material. Moreover, preferably, the bag 5 is also flexible.

In Figure 1 the containment envelope 2 is not shown.

In an alternative embodiment, the flexible bag 5 may be substituted with a different object, again porous, which is rigid or semi-rigid (for example a containment shell).

Use of the device 1 according to the first embodiment comprises extracting the bag 5 (or the shell, or the other object used) from the protective envelope 2, and immersing it in the liquid in which purification by removing the interfering factors must be carried out, or in which the sample to be purified is immersed.

In the second and in the third embodiments (Figures 2 to 5), the device 1 also comprises at least one container 6 inserted in the envelope 2, designed to contain the sample from which the interfering factors must be removed and in which the product 4 for removing the interfering factors is housed.

Figures 2 and 3 show the second embodiment of the device 1 which uses the containment element 3 of the first embodiment in a more complete device 1. In fact, in this case, inside the envelope 2 there is not only the container 6 (in the form of a jar) but also a lid 7 removably couplable to the container 6 for sealing it closed and together with the container forming a containment chamber 8. In contrast, the containment element 3 is in turn inserted in the chamber 8.

In this case, at the time of use, the container 6 and the lid 7 are extracted from the protective envelope 2, the lid 7 is uncoupled from the container 6 and the latter is filled with the liquid to be purified until it covers the containment element 3. The lid 7 is then coupled to the container 6 again.

It should be noticed that both the first embodiment and the second embodiment of this invention are suitable for the purification both of samples in the liquid state and samples in the solid state.

Whilst in the former case the sample is the self-same liquid in which the containment element 3 is immersed, in the latter case an operating liquid must be used, in which both the sample and the containment element 3 must be immersed.

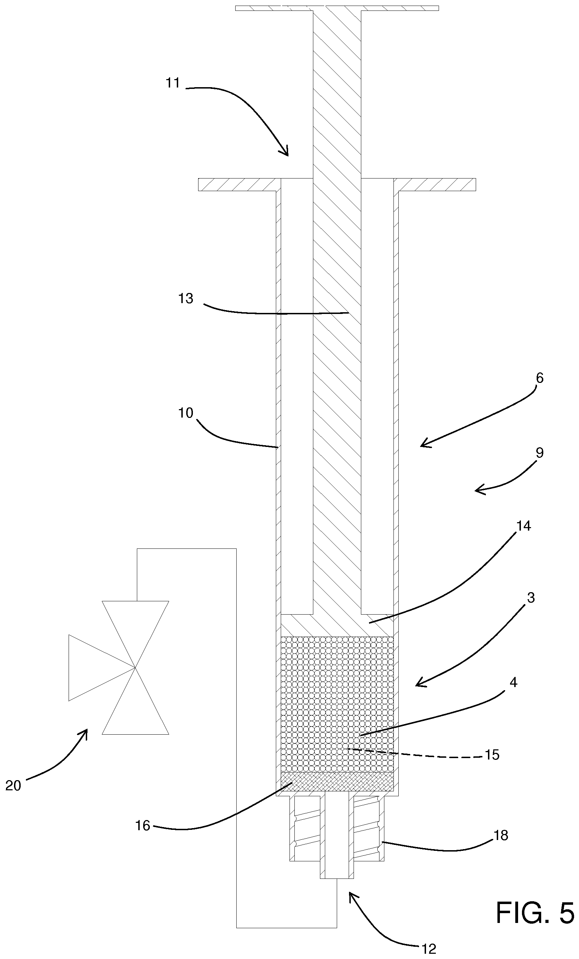

The third embodiment, illustrated in Figures 4 and 5, is different. This is intended exclusively for use in the purification of liquids by removing interfering factors.

In fact, in this case, inside the envelope 2 the device 1 comprises a syringe 9 which forms both the container 6 and the containment element 3.

In more detail, the syringe 9 comprises an external jacket 10 having an open first end 11 and an open second end 12. The open second end 12 forms a nozzle for sucking in and dispensing liquids, whilst a plunger 13 is inserted in the jacket 10 through the open first end 11. The plunger 13 comprises a head 14 slidably coupled in a sealed fashion to the inside of the jacket 10, and between the head 14 of the plunger 13 and the jacket 10 there is a variable-volume containment compartment 15 (its volume varying depending on the position of the head 14 of the plunger 13). This containment compartment 15 is also in fluid communication with the open second end 12 but only through a filter 16 which forms the porous portion of the containment element 3. In fact, according to this embodiment, the containment element 3 is formed by the jacket 10 (or more precisely by the portion of jacket 10 between the position of the head 14 of the plunger 13 and the filter 16), the head 14 of the plunger 13 and the filter 16. Consequently, the product 4 for removing interfering factors is inserted in the containment compartment 15.

Therefore, with this embodiment the liquid sample to be purified by removing interfering factors is sucked into the containment compartment 15 and expelled once the treatment is complete.

As shown in the accompanying drawings, according to the third embodiment of the invention, the device 1 may also comprise a suction and/or delivery needle/cannula 17 removably coupled to the second end 12 (for example by a Luer connector 18 as shown in the accompanying drawings). Moreover, advantageously, the second end 12, before use can be closed with a removable cap 19 (for example fixed to the Luer connector 18).

In a further alternative embodiment, the device 1 may also comprise a three-way valve 20 also removably couplable, with one of its ways (connectors), to the second end 12 (for example by means of the Luer connector 18). The other two ways of the valve 20 being able to be used one for suction of the fluid (if necessary subject to connection of a needle/a cannula 17) and the other for dispensing the fluid which has been decontaminated by removal of the interfering factors, to avoid the risks of recontamination during dispensing after the use of a single connection for suction and dispensing.

As already indicated, this invention also relates to a method for removing interfering factors from a sample to be subjected to microbiological examination, in which a device 1 according to any of the preceding claims is used. Advantageously, the sample may be either a processing liquid for an organ, tissue or cells intended for transplantation, or a sample of an organ, tissue or cells intended for transplantation, or a bodily fluid taken from a person, or a medicine, or a substance derived from humans. The term processing liquid means a fluid previously used to treat an organ, a tissue or cells, such as a preservation fluid or a decontamination fluid.

In general, the method requires that if the sample is liquid it must be put in fluid communication with the product 4 through the porous portion (for example either by immersing the containment element 3 in the sample, or by sucking the sample into the syringe 9 as described above relative to the use of the three embodiments illustrated in the accompanying drawings), and kept there for a first period of time. If, in contrast, the sample is solid, the method requires it to be immersed in an operating liquid which is in turn put in fluid communication with said product 4 through the porous portion (in this case the containment element 3 is advantageously immersed in the operating liquid), and kept there for a predetermined second period of time (usually longer than the first period of time, the other conditions being equal, since the interfering factors must be allowed to come out of the sample). Depending on requirements, the method may either require the use of a single device 1 for the treatment, with a relatively long predetermined treatment time, or the use of several devices one after another, each for a shorter time. Moreover, in the latter case, in general the total treatment time is less than or equal to that of the former case, the results being equal.

As already indicated, the method for removing interfering factors according to this invention is intended for use as a preliminary process before a microbiological examination, for example performed using the above-mentioned known equipment. In fact, thanks to this invention, what is subjected to the microbiological examination is not the sample (liquid or solid) as such, as was the case until now, but the sample purified by removing the interfering factors from it. Considering that, the sample may also at that point be inserted in vials without resins which therefore have a much more limpid culture liquid (the resins used in the vials today in fact tend to cause cloudiness). Thanks to this invention it is therefore possible to significantly increase the sensitivity even of prior art equipment.

With reference to the transplant sector, several preferred methods for removing interfering factors have also been provided.

A first example relates to corneas intended for transplantation. In this case the method comprises carrying out the following steps in asepsis (a method particularly suited to a device 1 such as the Syringe Resep described below):
- sterile removal of the syringe 9 and the needle 17 from the envelope 2;
- removal of the cap 19 from the syringe 9 and sterile screwing of the needle 17 onto the second end 12;
- suction of between 2 and 4 ml of liquid to be analysed for each gram of product 4 for removing interfering factors present in the device 1;
- agitation of the syringe 9 (preferably inverting it several times);
- leaving at ambient temperature for 15/20 minutes to promote removal of the interfering factors;
- expulsion of the liquid directly into the vial for microbiological analyses (such as a BACTEC bottle).

A second example relates to the sector of tissues intended for transplantation. In this case the method comprises carrying out the following steps in asepsis (a method particularly suited to a device 1 such as the Syringe Resep described below):
- sterile removal of the syringe 9 and the needle 17 from the envelope 2;
- removal of the cap 19 from the syringe 9 and sterile screwing of the needle 17 onto the second end 12;
- suction of between 3 and 5 ml of liquid to be analysed for each gram of product 4 for removing interfering factors present in the device 1;
- agitation of the syringe 9 (preferably inverting it several times);
- leaving at ambient temperature for 15/20 minutes to promote removal of the interfering factors;
- expulsion of the liquid directly into the vial for microbiological analyses (such as a BACTALERT bottle, flacon with culture broth).

In both of these examples the liquid to be examined may be the transportation liquid, the decontamination liquid, the washing liquid, a preservation or cryopreservation liquid.

Finally, it should be noticed that the context of this invention also covers cases in which removal applies to most but not all of the interfering factors.

### EXPERIMENTAL RESULTS

Below is a description of the results of removal of the interfering factors obtained using two different devices made in accordance with this invention.

### A. Device according to the third embodiment (hereinafter called Syringe Resep)

The device tested comprised a 10 ml polypropylene syringe 9 with a polypropylene filter 16 having 120 µm porosity, in which there were 0.5 g of a first resin consisting of Amberlite™ FPC3500, and 0.5 g of a second resin consisting of Amberlite™ XAD™18. Both of the resins had been previously activated and contained respectively 53% and 47% water, by weight.

A series of validation tests were carried out on said device.

### A.1. ASSESSMENT OF SYRINGE RESEP DEVICE PERFORMANCE ON LIQUIDS FOR THE DECONTAMINATION OF TISSUES

The Syringe Resep device underwent a series of tests to check its effective reliability in the removal of interfering factors from the processing liquid for tissues called BASE.128™.

The BASE 128™ liquid is a solution for the decontamination of human tissues intended for transplantation, produced and marketed by Al.Chi.Mi.A. S.r.l. with registered office in Ponte S.Nicolò (Padua province), Italy.

In terms of its composition, BASE 128™ comprises purified water, RPMI 1640 w L-Glutamine w/o NaHCO, sodium bicarbonate, HEPES buffer

(powder), cefotaxime sodium salt, gentamicin sulphate, vancomycin hydrochloride, amphotericin b deoxycholate and sodium pyruvate.

### A.1.1 Kinetics of removal of the antibiotic content of 5 ml of BASE.128™ by treatment with a Syringe Resep device

The kinetics of reduction of the antibiotic residue in 5 ml of BASE. 128™ by the Syringe Resep device were assessed. The result was that 20 minutes of sample incubation in the Syringe Resep device allow almost complete removal of the antibiotic content present in 5 ml of BASE. 128™.

The following are the results of the chromatographic analyses carried out using the HPLC technique. Each condition was subject to triple testing (that is to say, with three different prototypes of the Syringe Resep device) and each sample was double tested using HPLC.

**Table 1 Removal of antibiotic residues from samples of BASE.128™ (5ml).**

| **Treatment time with ResEP (min)** | **5** | **10** | **20** | **30** | **60** |
|---|---|---|---|---|---|
| % removal of vancomycin HCl | 100 | 100 | 100 | 100 | 100 |
| % removal of cefotaxime sodium salt | 100 | 100 | 100 | 100 | 100 |
| % removal of gentamicin sulphate | 88 | 94 | 96 | 97 | 100 |
| % removal of amphotericin b deoxycholate | 100 | 100 | 100 | 100 | 100 |

### A.1.2 Preliminary tests on the kinetics of removal of the antibiotic content of 10 ml of BASE.128™ by treatment with a Syringe Resep device.

The kinetics of reduction of the antibiotic residue in 10 ml of BASE. 128™ by the Syringe Resep device were assessed. The result was that in this case 20 minutes of sample incubation still allow good removal of the antibiotics, but longer times are needed to achieve better removal (more than 90% of the initial content). All of the analyses were carried out using HPLC.

The following are the results of the analyses carried out using HPLC. Each condition was subject to single testing (with only one prototype of the device) whilst each sample was double tested using HPLC.

**Table 2 Removal of antibiotic residues from samples of BASE.128™ (10ml).**

| **Treatment time with ResEP (min)** | **5** | **15** | **20** | **30** | **45** | **60** | **90** | **1380** |
|---|---|---|---|---|---|---|---|---|
| % removal of vancomycin HCl | 72 | 88 | 92 | 94 | 98 | 97 | 98 | 100 |
| % removal of cefotaxime sodium salt | 73 | 90 | 94 | 96 | 98 | 99 | 99 | 100 |
| % removal of gentamicin sulphate | 79 | 93 | 96 | 96 | 97 | 100 | 100 | 100 |
| % removal of amphotericin b deoxycholate | 64 | 89 | 91 | 93 | 93 | 94 | 97 | 100 |

In light of the results achieved, the Syringe Resep device tested is therefore considered suitable for treating volumes of liquid of between 1.5 ml and 5 ml to guarantee a good result in shorter times.

### A.1.3 Validation of the performance of the Syringe Resep device in collaboration with the cardiovascular tissue banks of Emilia Romagna

### (BTCER) and Lombardy (BTCL)

This study was carried out with the Applicant collaborating with the tissue banks indicated, and involved the cardiovascular tissue banks of Emilia Romagna (Italy) and of Lombardy (Italy) processing and decontaminating ten tissues (suitable and not suitable for transplantation) using the liquid BASE. 128™, subjecting to tests to check for microbiological negativity three separate liquids which came into contact with the tissues: the transportation liquid used before decontamination, the decontamination liquid and the cryopreservation liquid used after decontamination.

In particular, the treatment required the samples to be subjected to the following protocol:
- Decontamination: tissue incubation in the BASE.128™ solution using 25 ml of BASE.128™ for every gram of tissue, under the following time and temperature conditions:
   - 72 h at 4°C for vessels
   - 24 h at 4°C for heart valves
- Washing: after decontamination, the tissue was immersed in 25 ml of washing solution for every gram of tissue (BTCL: The tissues intended for transplantation were washed with "manual" agitation under a hood for 2-3 minutes at ambient temperature; BTCER: The tissues were washed by briefly rinsing with a change of washing liquid under a hood at ambient temperature).
- Cryopreservation: the tissue was immersed in approximately 100 ml of cold cryopreservation solution. The bag was sealed and placed in an automated cryofreezer with a cooling speed of -1 °C/min.

Moreover, tests to check for microbiological negativity were carried out both on samples of liquids treated according to the tissue bank standard method and on samples of liquid treated with the Syringe Resep device. The protocol for use of the Syringe Resep device required the liquid sample to be sucked up by means of the device to the "6 ml" mark (corresponding to a volume of 5 ml sucked up), briefly agitated and left to act for approximately 20 minutes. When that time had elapsed, the treated sample was inserted in the microbiological analysis equipment.

The tests carried out to check for microbiological negativity revealed various contaminations for the transportation liquids, and microbiological negativity for the washing and cryopreservation liquids.

Then, both the washing liquid and the cryopreservation liquid underwent HPLC assessment of the antibiotic residues.

The results in the two tissue banks are shown in the tables below:

**Table 3 Removal of antibiotic content by means of Syringe Resep on samples of processing liquids from BTCL**

| **Antibiotic** | **Sample treated** | **Final antibiotic residue present (µg/ml)** | **% antibiotic residue removal** |
|---|---|---|---|
| Vancomycin HCl | Decontamination liquid | 6.2 | 89 |
| | Washing liquid | <0.51* | 100 |
| Cefotaxime sodium salt | Decontamination liquid | 0.8 | 97 |
| | Washing liquid | <0.16* | 100 |
| Gentamicin sulphate | Decontamination liquid | 21.6 | 79 |
| | Washing liquid | < 1.71* | 100 |
| Amphotericin B deoxycholate | Decontamination liquid | 0.6 | 96 |
| | Washing liquid | < 0.44* | 100 |

| | | | |
|---|---|---|---|
| *instrument quantifiability limit | | | |

**Table 4 Removal of antibiotic content by means of Syringe Resep on samples of processing liquids from BTCER (*instrument quantifiability limit)**

| **Antibiotic** | **Sample treated** | **Final antibiotic residue present (µg/ml)** | **% antibiotic residue removal** |
|---|---|---|---|
| Vancomycin HCl | Decontamination liquid | 8.8 | 79 |
| | Cryopreservation liquid | 1.6 | 73 |
| Cefotaxime sodium salt | Decontamination liquid | 1.3 | 85 |
| | Cryopreservation liquid | <0.16* | 100 |
| Gentamicin sulphate | Decontamination liquid | 28.9 | 77 |
| | Cryopreservation liquid | < 1.71 * | 100 |
| Amphotericin B deoxycholate | Decontamination liquid | < 0.44* | 100 |
| | Cryopreservation liquid | < 0.44* | 100 |

| | | | |
|---|---|---|---|
| *instrument quantifiability limit | | | |

In the samples treated with the Syringe Resep device, a reduction in antibiotic residues of between 76% and 100% was therefore observed.

It should also be noticed that the reduced performance of the device, in the cases of vancomycin and gentamicin, was due to the presence of very "dirty" liquids (full of erythrocytes and other material derived from the treatment of the tissues). In contrast, further tests not shown demonstrated that, where the sample was difficult to treat, the use of two passes in a Syringe Resep (or use of two devices one after another with the methods indicated above) allowed the problem to be overcome.

In any case, tests carried out have shown that the quantity of residues encountered after treatment with the Syringe Resep device, then diluted in the device for the test to check for microbiological negativity, does not interfere with bacterial growth and that therefore the sample may be considered purified in terms of removal of interfering factors.

### A.1.4 Validation of the Syringe Resep device on samples from a study in collaboration with the Verona Skin Bank

This is a study carried out in collaboration with the Skin Bank of Verona, Italy. The aim of this study was to set up a suitable decontamination time/temperature protocol for skin tissues.

For that purpose, five tissues were decontaminated with BASE.128™ according to two protocols (100 ml at 37°C for 6h;100 ml at 22°C for 24h).

The samples of liquid and tissue were tested for microbiological negativity both by the Verona Skin Bank using its own standard method (which uses culture media) and by the Applicant. Only the liquid samples tested by the Applicant were treated with the Syringe Resep to remove interfering factors. Some of these samples were also assessed using HPLC to detect any antibiotic residues.

Double HPLC analysis carried out on 24 samples after treatment with the Syringe Resep device, showed complete removal of antibiotic residues present in the samples decontaminated with both protocols (again in this case, tests were run for Vancomycin, Cefotaxime, Gentamicin and Amphotericin B deoxycholate).

Comparison of the results obtained with the use of the Syringe Resep devices on one hand, and with the Skin Bank standard procedure on the other, also showed approximately 50% of false negatives in the results obtained using the standard procedure.

An interesting piece of data was obtained by also subjecting several samples of transportation liquids for the tissues not yet processed (therefore in theory free of antibiotic residues) to the action of the Syringe Resep device.

Some of these tested positive for microbiological contamination only after treatment with the devices, thus indicating the presence of interfering factors already in the tissues.

### A.2. ASSESSMENT OF THE PERFORMANCE OF THE RESEP DEVICE SYRINGE RESEP ON CORNEA PRESERVATION LIQUIDS

The effectiveness of the Syringe Resep device was also tested relative to two liquids for the preservation of corneas called CARRY-C™ and TISSUE- C™, also produced and marketed by Al.Chi.Mi.A. S.r.l. with registered office in Ponte S.Nicolò (Padua province), Italy.

In terms of composition, TISSUE-C™ comprises purified water, Penicillin, Streptomycin, Amphotericin B, MEM (powder), NEW BORN CALF SERUM, sodium pyruvate and sodium bicarbonate, whilst CARRY-C™ has the same ingredients plus dextran T500.

The assessments are still in progress.

### A.2.1 Kinetics of removal of streptomycin and penicillin G

Preliminary tests were carried out on 5 ml samples of CARRY-C™ and Tissue-C™ and the removal of Penicillin G and Streptomycin was assessed using HPLC.

An assessment was also made of how performance varied depending on the total grams of product 4 for removing interfering factors used in the device (the resins used and the ratios of them by weight being equal), giving the following indications:

**Table 5 Removal of antibiotic content by means of Syringe Resep on samples of cornea preservation liquids**

| | **Tissue-C™** | | **CARRY-C™** | |
|---|---|---|---|---|
| **Treatment (grams * minutes)** | **% streptomycin removal** | **% penicillin G removal** | **% streptomycin removal** | **% penicillin G removal** |
| 1g*20' | 64.86 | 100 | 67.96 | 95.74 |
| 1g*30' | 73.28 | 100 | 72.66 | 95.67 |
| 1g*45' | 79.25 | 100 | 79.60 | 95.26 |
| 1g*60' | 79.34 | 100 | / | / |
| 1.2g*20' | 64.47 | 100 | / | / |
| 1.4g*20' | 66.86 | 100 | / | / |
| 1.4g*30' | 77.82 | 100 | / | / |
| 1.4g*45' | 79.64 | 100 | / | / |
| 2g*20 | 73.59 | 100 | / | / |

As shown by the results, there was almost total removal of Penicillin G, whilst removal was only partial in the case of streptomycin.

However, preliminary tests indicate that, by passing the liquids twice, one time after another, in Syringe Resep devices, even streptomycin can be completely removed. In particular, 99% of streptomycin can be removed using two Syringe Resep devices one after another, keeping the liquid for ten minutes in each device.

All of these analysis were performed on individual samples which were double tested using HPLC.

### A.2.2 Validation of prototypes with the Monza Eye Bank (BOM)

Validation is in progress of the Syringe Resep device on the liquids used for the preservation and decontamination of cornea tissues at BOM (preservation liquids used: Tisssue-C, CARRY-C™ and Eusol-C also produced and marketed by AI.Chi.Mi.A. S.r.l.).

These tests involve a comparison of the result of the test to check for microbiological negativity for samples subjected to treatment with the Syringe Resep, with that for samples subjected to the BOM standard procedure.

The tests are still in progress. However, up to now, using the device in accordance with the invention, 20% of false negatives have been identified.

### A3. ASSESSMENT OF INTERACTIONS BETWEEN SYRINGE RESEP DEVICE COMPONENTS AND STRAINS OF BACTERIA

To assess any interactions between the components of the device and potential contaminants present in the samples to be treated, tests were carried out with the strains of bacteria indicated by the Pharmacopoeia (S. aureus ATCC6538, P. aeruginosa ATCC9027, C. albicans ATCC10231, B. subtilis ATCC6633, A. brasiliensis ATCC16404, C. sporogenes ATCC19404) to check the possibility of completely recovering what was previously inoculated.

For each strain of bacteria between 1 and 10 CFU were inoculated in samples of liquid consisting of between 1.5 and 3 ml. In all of the cases after treatment with the Syringe Resep device it was possible to recover all of the CFU inoculated.

Each condition was triple tested and compared with corresponding double tested controls (inoculation in syringes without resins and filter 16 and inoculation in test tube).

It may therefore be said that there is no interaction between the components of the device and the strains of bacteria tested. The Syringe Resep device therefore does not interfere with the recovery of any contaminants present in the samples.

An additional test on S. aureus also indicates that even passing twice in Syringe Resep devices does not affect bacterial recovery of 1-10 CFU.

### B. Device according to the second embodiment of this invention (hereinafter called TISSUE RESEP)

The device tested comprises a 15 ml polypropylene container 6 in which a PET bag 5 is inserted consisting of a regular mesh with holes having a 190 µm diagonal, containing 0.75 g of a first resin consisting of Amberlite™ FPC3500, and 0.75 g of a second resin consisting of Amberlite™ XAD™18. As in the previous case, both of the resins had been previously activated and contain respectively 53% and 47% water, by weight.
A series of validation tests were and are being carried out on said device.

### B.1. ASSESSMENT OF THE PERFORMANCE OF THE TISSUE RESEP DEVICE ON SAMPLES OF TISSUES DECONTAMINATED WITH BASE. 128™

Initial tests for the removal of antibiotic residues contained in tissues were carried out on homogenized material consisting of cardiovascular tissues (approximately 0.5 g of tissue) decontaminated for 72 h at 4°C in BASE.128™ (with the proportion of 25 ml of liquid per gram of tissue).

After placing the homogenized material and the liquid in the Tissue Resep, the latter was agitated respectively for 30 or 60 minutes at ambient temperature.

The test results were as follows:

**Table 6 Removal of antibiotic content using tissue ResEP on homogenized material consisting of cardiovascular tissues decontaminated for 72h/4°C in BASE. 128™.**

| **Residue removal** | **Tissue treated 30'/A.T. agitated** | **Tissue treated 60'/A.T. agitated** |
|---|---|---|
| Vancomycin HCl (%) | 74 | 89 |
| Cefotaxime sodium salt (%) | 100 | 100 |
| Gentamicin sulphate (%) | 74 | 96 |
| Amphotericin B deoxycholate (%) | 100 | 100 |

Cefotaxime and amphotericin were completely removed. In the case of vancomycin, approximately 89% had been removed after 1 h of treatment of the tissue with the device. In contrast, 96% of gentamicin was removed in the same conditions. Reducing the application time to 30' reduced performance. These analysis were performed on 3 tissues which were double tested using HPLC.

### B.2. ASSESSMENT OF INTERACTIONS BETWEEN DEVICE COMPONENTS AND STRAINS OF BACTERIA

Initial assessments of interactions between device components and strains of bacteria were carried out on the strain S. aureus. In this case too, recovery of 1-10 CFU was confirmed. Further tests are in progress on the strains indicated by the Pharmacopoeia.

This invention brings important advantages.

In fact, the device and method provided allow the sample which must be subjected to microbiological examinations to be treated in such a way that the subsequent examinations can be carried out with results which are much more reliable than is currently the case.

Secondly, with reference to the transplant sector, this invention makes it possible to also detect bacterial contaminations which until now would have given false negatives.

Furthermore, if applied to examinations of biological fluids, as well as guaranteeing greater sensitivity this invention may allow a significant reduction in the quantity of sample needed in order to carry out the analysis, with particular benefits in the paediatric field.

Moreover, in general, all other aspects being equal, preliminary application of this invention allows a reduction in the times required by prior art equipment to detect a microbiological contamination.

Finally, it should be noticed that this invention is relatively easy to produce and that even the cost linked to implementing the invention is not very high.

Moreover, all details of the invention may be substituted with other technically equivalent elements and the materials used, as well as the shapes and dimensions of the various components, may vary according to requirements.

## Claims

1. A device for removing interfering factors from samples to be subjected to microbiological examination, said interfering factors being any substance able to inhibit or slow bacterial reproduction on which microbiological examination of the samples is based,
**characterized in that**
the device is ready to use,
the device comprising a sealed protective envelope (2) and a containment element (3),
the containment element (3) being positioned inside the envelope (2) and containing at least one product (4) for removing interfering factors, the product (4) being in the form of granules, the particle size of the product (4) having a nominal size and a certain variation range,
the containment element (3) being closed and comprising at least one porous portion that is permeable to liquids and to the interfering factors, said at least one porous portion having a porosity with dimensions less than the nominal size, such that said at least one porous portion allows the passage of particles of product (4) having dimensions less than a minimum size which is indicated as a percentage of the nominal size, and such that said at least one porous portion retains the other granules of product (4),
everything contained in the envelope (2) being sterile and the envelope (2) being such that it prevents the passage of contaminants through it in order to preserve the sterile condition of everything contained in the envelope (2), wherein the containment element (3) is extracted or sterilely removed from the envelope (2) at the time of use.

2. The device according to claim 1, the device being intended for carrying out a preliminary process before a microbiological examination that is intended either to verify the microbiological negativity of a sample or to measure a degree and type of microbiological contamination of the sample, said preliminary process allowing to provide the microbiological examination with a sample purified of interfering factors.

3. The device according to claim 1 or 2, also comprising a container (6) for containing the sample from which the interfering factors must be removed, the container (6) being inserted in the envelope (2) and containing said product (4).

4. The device according to claim 1, 2 or 3, wherein the containment element (3) is a containment bag (5) made of porous material.

5. The device according to claim 3, also comprising a lid (7) removably couplable to the container (6), for sealing it closed and together with the container forming a containment chamber (8), both being inserted in the protective envelope (2), the containment element (3) being inserted in said chamber (8).

6. The device according to claim 5, wherein the containment element (3) is a containment bag (5) made of porous material.

7. The device according to claim 3, wherein the container (6) is a syringe (9) comprising an external jacket (10) having an open first end (11) and an open second end (12), the open second end (12) forming a nozzle for sucking in and dispensing liquids, and a plunger (13) inserted in the jacket (10) through the open first end (11) and comprising a head (14) slidably coupled in a sealed fashion to the inside of the jacket (10), between the head (14) of the plunger (13) and the jacket (10) there being a variable-volume containment compartment (15) which is in fluid communication with the open second end (12) through a filter (16) which forms said porous portion, the product (4) for removing interfering factors being inserted in the containment compartment (15), and the containment element (3) being formed by the jacket (10), the head (14) of the plunger (13) and the filter (16).

8. The device according to any one of the preceding claims, **characterized in that** the product (4) is a composition comprising a mixture of a first substance and a second substance, both in the form of granules, the first substance in turn comprising a first resin belonging to the ion exchange resins family, and the second substance in turn comprising a second resin belonging to the non-ionic hydrophobic resins family, the first substance and the second substance being present in a ratio by weight of between 0.5 and 2.

9. The device according to claim 8, **characterized in that** at least the second substance is hydrated and comprises a quantity of water equal to at least 30% of its own weight.

10. The device according to claim 8 or 9, **characterized in that** the first substance and the second substance are present in a ratio by weight of between 0.8 and 1.25.

11. The device according to claim 8, 9 or 10, **characterized in that** the first substance is hydrated and comprises a quantity of water equal to at least 50% of its own weight and/or the second substance is hydrated and comprises a quantity of water equal to at least 46% of its own weight.

12. The device according to claim 11, **characterized in that** the first substance comprises a quantity of water not greater than 67% of its own weight and/or the second substance comprises a quantity of water not greater than 63% of its own weight.

13. The device according to any one of the claims from 8 to 12, **characterized in that** the first resin is methacrylate-divinylbenzene based and/or the second resin is polystyrene-divinylbenzene based.

14. A method for removing interfering factors from a sample to be subjected to microbiological examination, said interfering factors being any substance able to inhibit or slow bacterial reproduction on which microbiological examination of the sample is based,
**characterized in that** it uses at least one device according to any of the preceding claims, the method including the steps of:
- extracting or sterilely removing the containment element (3) from the envelope (2);
- if the sample is liquid, putting the sample in fluid communication with the product (4) through the porous portion of the containment element (3) for a first period of time; or respectively
- if the sample is solid, immersing the sample in an operating liquid and putting the operating liquid in fluid communication with the product (4) through the porous portion of the containment element (3) for a second period of time.

15. The method according to claim 14, wherein a plurality of said devices is used one after another for removing the interfering factors in two or more steps one after another.

16. The method according to claim 14 or 15, wherein the sample is either a processing liquid for an organ, tissue or cells intended for transplantation, or a sample of an organ, tissue or cells intended for transplantation, or a bodily fluid taken from a living subject, or a medicine, or a substance derived from humans.

17. The method according to any of claims 14 to 16, wherein the device is according to claim 4 or 6 and the step of putting the sample or respectively the operating liquid in fluid communication with the product (4) is carried out by immersing the containment bag (5) in the sample or respectively in the operating liquid.

18. The method according to any of claims 14 to 16, wherein the device is according to claim 5 or 6 and the step of putting the sample or respectively the operating liquid in fluid communication with the product (4) is carried out by the sub-steps of:
- uncoupling the lid (7) from the container (6);
- filling the container (6) with the sample or respectively with the operating liquid until it covers the containment element (3); and
- coupling the lid (7) to the container (6) again.

19. The method according to any of claims 14 to 16, wherein the device is according to claim 7 and the step of putting the sample in fluid communication with the product (4) is carried out by sucking the sample into the syringe (9).

## Patentansprüche

1. Vorrichtung zum Entfernen störender Faktoren aus Proben, die einer mikrobiologischen Untersuchung unterzogen werden, wobei die genannten störenden Faktoren aus jeder Substanz bestehen, die in der Lage ist, die bakterielle Reproduktion zu verhindern oder zu verlangsamen, auf welcher die mikrobiologische Untersuchung der Proben basiert, **dadurch gekennzeichnet, dass** die Vorrichtung gebrauchsfertig ist, die Vorrichtung ein abgedichtetes Schutzgehäuse (2) und ein Aufnahmeelement (3) enthält, wobei das Aufnahmeelement (3) im Inneren des Gehäuses (2) angeordnet ist und wenigstens ein Produkt (4) zum Entfernen störender Faktoren enthält, wobei das Produkt (4) in Form eines Granulates ist, wobei die Partikelgrösse des Produktes (4) eine nominale Grösse hat und einen gewissen Abweichungsbereich enthält, wobei das Auf nahmeelement (3) verschlossen ist und wenigstens einen porösen Abschnitt enthält, der durchlässig für Flüssigkeiten und für die störenden Faktoren ist, wobei der wenigstens eine poröse Abschnitt eine Porosität von Abmessungen aufweist, die geringer sind als die nominale Grösse, und zwar solche, dass der genannte wenigstens eine poröse Abschnitt das Durchlassen von Partikeln des Produktes (4) erlaubt, die geringere Abmessungen haben als eine Mindestgrösse, welche als ein Prozentsatz der nominalen Grösse angegeben ist, und solche, dass der genannte wenigstens eine poröse Abschnitt die anderen Körner des Produktes (4) zurückhält, wobei alles was in dem Gehäuse (2) enthalten ist, steril und das Gehäuse (2) ein solches ist, dass das Durchtreten von kontaminierenden Substanzen durch dieses verhindert wird, um den sterilen Zustand von allem, was in dem Gehäuse (2) enthalten ist, beibehalten zu können, wobei das Aufnahmeelement (3) aus dem Gehäuse (2) zum Benutzungszeitpunkt herausgezogen oder steril entfernt wird.

2. Vorrichtung nach Patentanspruch 1, wobei die Vorrichtung dafür gedacht ist, einen vorhergehendes Verfahren vor einer mikrobiologischen Untersuchung durchzuführen, dazu bestimmt, entweder die mikrobiologische Negativität einer Probe festzustellen oder um Grad und Typ einer mikrobiologischen Kontamination der Probe zu messen, wobei es das genannte vorhergehende Verfahren erlaubt, die mikrobiologische Untersuchung mit einer von störenden Faktoren gereinigten Probe durchzuführen.

3. Vorrichtung nach Patentanspruch 1 oder 2, ebenfalls enthaltend einen Behälter (6) zur Aufnahme der Probe, aus welcher die störenden Faktoren entfernt werden müssen, wobei der Behälter (6) in das Gehäuse (2) eingesetzt wird und das genannte Produkt (4) enthält.

4. Vorrichtung nach Patentanspruch 1, 2 oder 3, bei welcher das Aufnahmeelement (3) aus einem Aufnahmebeutel (5) aus porösem Material besteht.

5. Vorrichtung nach Patentanspruch 3, ebenfalls enthaltend einen Deckel (7), der abnehmbar mit dem Behälter (6) verbindbar ist, um diesen dicht zu verschliessen und zusammen mit dem Behälter eine Aufnahmekammer (8) zu bilden, wobei beide in das Schutzgehäuse (2) eingesetzt werden, und wobei das Aufnahmeelement (3) in die genannte Kammer (8) eingesetzt wird.

6. Vorrichtung nach Patentanspruch 5, bei welcher das Auf nahmeelement (3) ein Aufnahmebeutel (5) aus porösem Material ist.

7. Vorrichtung nach Patentanspruch 3, bei welcher der Behälter (6) eine Spritze (9) ist, enthaltend einen äusseren Mantel (10) mit einem ersten offenen Ende (11) und einem zweiten offenen Ende (12), wobei das zweite offene Ende (12) eine Düse zum Aufziehen und Abgeben von Flüssigkeiten bildet, sowie einen Kolben (13), eingesetzt in den Mantel (10) durch das erste offene Ende (11) und enthaltend einen Kopf (14), der verschiebbar in abdichtender Weise an der Innenseite des Mantels (10) anliegt, wobei zwischen dem Kopf (14) des Kolbens (13) und dem Mantel (10) ein im Volumen veränderbarer Aufnahmeraum (15) vorhanden ist, welcher sich in Flüssigkeitsverbindung mit dem zweiten offenen Ende (12) befindet, und zwar durch einen Filter (16), welcher den genannten porösen Abschnitt bildet, wobei das Produkt (4) zum Entfernen störender Faktoren in den Aufnahmeraum (15) eingegeben ist, und wobei das Aufnahmeelement (3) durch den Mantel (10), den Kopf (14) des Kolbens (13) und den Filter (16) gebildet ist.

8. Vorrichtung nach einem jeden der vorstehenden Patentansprüche, **dadurch gekennzeichnet, dass** das Produkt (4) eine Zusammensetzung ist, enthaltend eine Mischung aus einer ersten Substanz und einer zweiten Substanz, beide in Form von Granulat, wobei die erste Substanz wiederum ein erstes Harz enthält, das zu der Familie der Ionenaustausch-Harze gehört, und die zweite Substanz wiederum ein zweites Harz enthält, das zu der Familie der nichtionischen, wasserabweisenden Harze gehört, wobei die erste Substanz und die zweite Substanz in einem Gewichtsverhältnis zwischen 0.5 und 2 vorhanden sind.

9. Vorrichtung nach Patentanspruch 8, **dadurch gekennzeichnet, dass** wenigstens die zweite Substanz hydriert ist und eine Wassermenge entsprechend wenigstens 30% ihres Eigengewichtes enthält.

10. Vorrichtung nach Patentanspruch 8 oder 9, **dadurch gekennzeichnet, dass** die erste Substanz und die zweite Substanz in einem Gewichtsverhältnis zwischen 0.8 und 1.25 vorhanden sind.

11. Vorrichtung nach Patentanspruch 8, 9 oder 10, **dadurch gekennzeichnet, dass** die erste Substanz hydriert ist und eine Wassermenge entsprechend wenigstens 50% ihres Eigengewichtes enthält, und/oder die zweite Substanz hydriert ist und eine Wassermenge entsprechend wenigstens 46% ihres Eigengewichtes enthält.

12. Vorrichtung nach Patentanspruch 11, **dadurch gekennzeichnet, dass** die erste Substanz eine Wassermenge enthält, die nicht grösser ist als 67% ihres Eigengewichtes, und/oder die zweite Substanz eine Wassermenge enthält, die nicht grösser ist als 63% ihres Eigengewichtes.

13. Vorrichtung nach einem jeden der Patentansprüche von 8 bis 12, **dadurch gekennzeichnet, dass** das erste Harz auf Methacrylat-Butadienbenzen basiert, und/oder das zweite Harz auf Polystyrol-Butadienbenzen basiert.

14. Verfahren zum Entfernen von störenden Faktoren aus einer Probe, die einer mikrobiologischen Untersuchung unterzogen werden soll, wobei die genannten störenden Faktoren aus jeder Substanz bestehen, die in der Lage ist, die bakterielle Reproduktion zu verhindern oder zu verlangsamen, auf welcher die mikrobiologische Untersuchung der Proben basiert, **dadurch gekennzeichnet, dass** es wenigstens eine Vorrichtung nach einem jeden der vorstehenden Patentansprüche verwendet, wobei das Verfahren die folgenden Schritte enthält:
- Herausziehen oder das sterile Entfernen des Aufnahmeelementes (3) aus dem Gehäuse (2);
- wenn die Probe flüssig ist, diese durch den porösen Abschnitt des Aufnahmeelements (3) für eine erste Dauer mit dem Produkt (4) in Flüssigkeitsverbindung bringen; beziehungsweise
- wenn die Probe fest ist, diese in eine Arbeitsflüssigkeit eintauchen und die Arbeitsflüssigkeit durch den porösen Abschnitt des Aufnahmeelements (3) für eine zweite Dauer mit dem Produkt (4) in Flüssigkeitsverbindung bringen.

15. Verfahren nach Patentanspruch 14, bei welchem eine Anzahl der genannten Vorrichtungen eine nach der anderen benutzt wird, um die störenden Faktoren in zwei oder mehreren Schritten einen nach dem anderen zu entfernen.

16. Verfahren nach Patentanspruch 14 oder 15, bei welchem die Probe entweder aus einer Behandlungsflüssigkeit für ein zur Transplantation bestimmtes Organ, Gewebe oder Zellen besteht, oder aus einer Probe eines zur Transplantation bestimmten Organs, Gewebes oder Zellen, oder aus einer Körperflüssigkeit eines lebenden Subjektes, oder aus einem Medikament oder einer Substanz menschlicher Herkunft.

17. Verfahren nach einem jeden der Patentansprüche von 14 bis 16, bei welchem die Vorrichtung jene nach Patentanspruch 4 oder 6 ist und der Schritt, mit welchem die Probe oder beziehungsweise die Arbeitsflüssigkeit mit dem Produkt (4) in Flüssigkeitsverbindung gebracht wird, durch das Eintauchen des Aufnahmebeutels (5) in die Probe oder beziehungsweise in die Arbeitsflüssigkeit erfolgt.

18. Verfahren nach einem jeden der Patentansprüche von 14 bis 16, bei welchem die Vorrichtung jene nach Patentanspruch 5 oder 6 ist und der Schritt, mit welchem die Probe oder beziehungsweise die Arbeitsflüssigkeit mit dem Produkt (4) in Flüssigkeitsverbindung gebracht wird, durch folgende Unterschritte ausgeführt wird:
- Lösen des Deckels (7) von dem Behälter (6);
- Befüllen des Behälters (6) mit der Probe oder beziehungsweise mit der Arbeitsflüssigkeit, bis diese das Auf nahmeelement (3) bedeckt; und
- das erneute Befestigen des Deckels (7) an dem Behälter (6).

19. Verfahren nach einem jeden der Patentansprüche von 14 bis 16, bei welchem die Vorrichtung jene nach Patentanspruch 7 ist und der Schritt, mit welchem die Probe in Flüssigkeitsverbindung mit dem Produkt (4) gebracht wird, durch das Aufziehen der Probe in eine Spritze (9) erfolgt.

## Revendications

1. Un dispositif pour éliminer les facteurs d'interférence d'échantillons à soumettre à un examen microbiologique, lesdits facteurs d'interférence étant toute substance en mesure d'inhiber ou de ralentir la reproduction bactérienne sur laquelle se base l'examen microbiologique des échantillons,
**caractérisé en ce que**
le dispositif est prêt à l'emploi,
le dispositif comprenant une enveloppe de protection scellée (2) et un élément de confinement (3),
l'élément de confinement (3) étant positionné à l'intérieur de l'enveloppe (2) et contenant au moins un produit (4) pour l'élimination des facteurs d'interférence, le produit (4) étant sous la forme de granules, la dimension particulaire du produit (4) ayant une taille nominale et une plage de variation donnée,
l'élément de confinement (3) étant fermé et comprenant au moins une portion poreuse qui est perméable aux liquides et aux facteurs d'interférence, ladite au moins une portion poreuse ayant une porosité dont les dimensions sont inférieures à la taille nominale, de sorte que ladite au moins une portion poreuse permet le passage de particules de produit (4) ayant des dimensions inférieures à une taille minimale qui est indiquée comme pourcentage de la taille nominale, et de sorte que ladite au moins une portion poreuse retient les autres granules de produit (4),
tout ce qui est contenu dans l'enveloppe (2) étant stérile et l'enveloppe (2) étant telle qu'elle empêche le passage de contaminants à travers elle afin de préserver la condition de stérilité de tout ce qui est contenu dans l'enveloppe (2),
où l'élément de confinement (3) est extrait ou enlevé de façon stérile de l'enveloppe (2) au moment de l'utilisation.

2. Le dispositif selon la revendication 1, tel dispositif étant prévu pour effectuer un processus préliminaire avant un examen microbiologique qui est destiné à vérifier la négativité microbiologique d'un échantillon ou à mesurer un degré et type de contamination microbiologique de l'échantillon, ledit processus préliminaire permettant de fournir à l'examen microbiologique un échantillon purifié des facteurs d'interférence.

3. Le dispositif selon la revendication 1 ou 2, comprenant aussi un récipient (6) pour contenir l'échantillon duquel les facteurs d'interférence doivent être éliminés, le récipient (6) étant inséré dans l'enveloppe (2) et contenant ledit produit (4).

4. Le dispositif selon la revendication 1, 2 ou 3, dans lequel l'élément de confinement (3) est un sac de confinement (5) réalisé dans un matériau poreux.

5. Le dispositif selon la revendication 3, comprenant aussi un couvercle (7) pouvant être accouplé de façon amovible avec le récipient (6), pour le fermer hermétiquement et définir avec ledit récipient une chambre de confinement (8), tous les deux étant insérés dans l'enveloppe de protection (2), l'élément de confinement (3) étant inséré dans ladite chambre (8).

6. Le dispositif selon la revendication 5, dans lequel l'élément de confinement (3) est un sac de confinement (5) réalisé dans un matériau poreux.

7. Le dispositif selon la revendication 3, dans lequel le récipient (6) est une seringue (9) comprenant une chemise extérieure (10) ayant une première extrémité ouverte (11) et une deuxième extrémité ouverte (12), la deuxième extrémité ouverte (12) formant une buse pour l'aspiration et la distribution de liquides, et un piston (13) inséré dans la chemise (10) à travers la première extrémité ouverte (11) et comprenant une tête (14) accouplée de façon coulissante de manière étanche à l'intérieur de la chemise (10), entre la tête (14) du piston (13) et la chemise (10) étant défini un compartiment de confinement à volume variable (15) qui est en communication de fluide avec la deuxième extrémité ouverte (12) à travers un filtre (16) qui forme ladite portion poreuse, le produit (4) pour l'élimination des facteurs d'interférence étant inséré dans le compartiment de confinement (15), et l'élément de confinement (3) étant formé par la chemise (10), la tête (14) du piston (13) et le filtre (16).

8. Le dispositif selon l'une quelconque des revendications précédentes, **caractérisé en ce que** le produit (4) est une composition comprenant un mélange d'une première substance et d'une deuxième substance, toutes deux sous la forme de granules, la première substance comprenant à son tour une première résine appartenant à la famille des résines échangeuses d'ions, et la deuxième substance comprenant à son tour une deuxième résine appartenant à la famille des résines hydrophobes non ioniques, la première substance et la deuxième substance étant présentes dans un rapport en poids compris entre 0,5 et 2.

9. Le dispositif selon la revendication 8, **caractérisé en ce qu'**au moins la deuxième substance est hydratée et comprend une quantité d'eau égale à au moins 30 % de son propre poids.

10. Le dispositif selon la revendication 8 ou 9, **caractérisé en ce que** la première substance et la deuxième substance sont présentes dans un rapport en poids compris entre 0,8 et 1,25.

11. Le dispositif selon la revendication 8, 9 ou 10, **caractérisé en ce que** la première substance est hydratée et comprend une quantité d'eau égale à au moins 50 % de son propre poids et/ou la deuxième substance est hydratée et comprend une quantité d'eau égale à au moins 46 % de son propre poids.

12. Le dispositif selon la revendication 11, **caractérisé en ce que** la première substance comprend une quantité d'eau qui n'est pas supérieure à 67 % de son propre poids et/ou la deuxième substance comprend une quantité d'eau qui n'est pas supérieure à 63 % de son propre poids.

13. Le dispositif selon l'une quelconque des revendications de 8 à 12, **caractérisé en ce que** la première résine est à base de méthacrylatedivinylbenzène et/ou la deuxième résine est à base de polystyrènedivinylbenzène.

14. Un procédé pour éliminer les facteurs d'interférence d'un échantillon à soumettre à un examen microbiologique, lesdits facteurs d'interférence étant toute substance en mesure d'inhiber ou de ralentir la reproduction bactérienne sur laquelle se base l'examen microbiologique de l'échantillon, **caractérisé en ce qu'**il utilise au moins un dispositif selon l'une quelconque des revendications précédentes, le procédé comprenant les phases consistant à :
- extraire ou enlever de façon stérile l'élément de confinement (3) de l'enveloppe (2) ;
- si l'échantillon est liquide, mettre l'échantillon en communication de fluide avec le produit (4) à travers la portion poreuse de l'élément de confinement (3) pendant une première période de temps ; ou respectivement
- si l'échantillon est solide, immerger l'échantillon dans un liquide de travail et mettre ledit liquide de travail en communication de fluide avec le produit (4) à travers la portion poreuse de l'élément de confinement (3) pendant une deuxième période de temps.

15. Le procédé selon la revendication 14, dans lequel une pluralité desdits dispositifs est utilisée l'un après l'autre pour éliminer les facteurs d'interférence en deux phases ou plus successives.

16. Le procédé selon la revendication 14 ou 15, dans lequel l'échantillon est soit un liquide de traitement pour un organe, tissu ou cellules destinés à une transplantation, soit un échantillon d'un organe, tissu ou cellules destinés à une transplantation, soit un fluide corporel prélevé d'un sujet vivant, soit un médicament, soit une substance de dérivation humaine.

17. Le procédé selon l'une quelconque des revendications de 14 à 16, dans lequel le dispositif est selon la revendication 4 ou 6 et la phase consistant à mettre l'échantillon ou respectivement le liquide de travail en communication de fluide avec le produit (4) est effectuée en immergeant le sac de confinement (5) dans l'échantillon ou respectivement dans le liquide de travail.

18. Le procédé selon l'une quelconque des revendications de 14 à 16, dans lequel le dispositif est selon la revendication 5 ou 6 et la phase consistant à mettre l'échantillon ou respectivement le fluide de travail en communication de fluide avec le produit (4) est effectuée au moyen des sous-phases consistant à :
- désaccoupler le couvercle (7) du récipient (6) ;
- remplir le récipient (6) avec l'échantillon ou respectivement avec le liquide de travail jusqu'à ce qu'il couvre l'élément de confinement (3) ; et
- accoupler à nouveau le couvercle (7) avec le récipient (6).

19. Le procédé selon l'une quelconque des revendications de 14 à 16, dans lequel le dispositif est selon la revendication 7 et la phase consistant à mettre l'échantillon en communication de fluide avec le produit (4) est effectuée en aspirant l'échantillon dans la seringue (9).
